(19) Europäisches Patentamt
European Patent Office
Office européen des brevets

(11) EP 0 776 879 A1

(12) EUROPÄISCHE PATENTANMELDUNG

(43) Veröffentlichungstag:
**04.06.1997 Patentblatt 1997/23**

(51) Int. Cl.$^6$: **C07C 41/28**, C07C 43/16

(21) Anmeldenummer: **96118612.9**

(22) Anmeldetag: **20.11.1996**

(84) Benannte Vertragsstaaten:
**AT BE CH DE FR GB IT LI NL**

(30) Priorität: **29.11.1995 DE 19544450**

(71) Anmelder: **BASF AKTIENGESELLSCHAFT**
**67056 Ludwigshafen (DE)**

(72) Erfinder:
- **Schröder, Jürgen, Dr.**
  **67071 Ludwigshafen (DE)**
- **Böck, Stefan, Dr.**
  **67069 Ludwigshafen (DE)**
- **Ebel, Klaus, Dr.**
  **68623 Lampertheim (DE)**

# (54) Verfahren zur Herstellung von Enolethern

(57) Verfahren zur Herstellung von Enolethern der allgemeinen Formel I

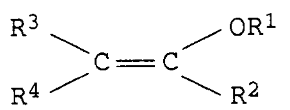

(I),

in der

| | |
|---|---|
| $R^1$,$R^2$,$R^3$,$R^4$,$R^5$ | $C_1$- bis $C_{20}$-Alkyl, $C_2$- bis $C_{20}$-Alkenyl oder $C_7$- bis $C_{20}$-Phenylalkyl, |
| $R^2$,$R^3$,$R^4$ | Wasserstoff, Aryl, Cyano, -COOR$^5$, durch -COOR$^5$, -C=O, Cyano, $C_1$- bis $C_{12}$-Alkoxy ein- bis dreifach substituiertes oder unterbrochenes $C_1$- bis $C_{12}$-Alkyl, durch $C_1$- bis $C_{12}$-Alkyl, $C_1$- bis $C_{12}$-Alkoxy, Halogen, Cyano ein- bis dreifach substituiertes Aryl oder $C_7$- bis $C_{20}$-Phenylalkyl und |
| $R^2$ und $R^4$ oder $R^3$ und $R^4$ | gemeinsam eine $C_3$- bis $C_{10}$-Alkylenkette oder gemeinsam eine $C_2$- bis $C_{20}$-Alkylidenkette |

bedeuten, aus Acetalen oder Ketalen der allgemeinen Formel II

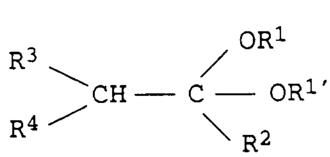

(II),

in der $R^1$, $R^2$, $R^3$ und $R^4$ die obengenannten und $R^{1'}$ unabhängig von $R^1$ dieselben Bedeutungen haben, in der Gasphase bei Temperaturen von 100 bis 550°C und einem Druck von 0,001 bis 5 bar an Heterogenkatalysatoren, indem man als Heterogenkatalysatoren hochporöse Oxide der II., III. und IV. Hauptgruppe oder der II. und IV. Nebengruppe des Periodensystems der Elemente oder deren Gemische mit einem pH-Wert entsprechend 7 bis 14, einer Porosität von 40 bis 80% und einer BET-Oberfläche von 0,5 bis 250 $m^2$/g einsetzt.

EP 0 776 879 A1

## Beschreibung

Die vorliegende Erfindung betrifft ein Verfahren zur Herstellung von Enolethern durch Umsetzung von Acetalen/Ketalen in der Gasphase an hochporösen oxidischen Heterogenkatalysatoren.

Aus der DE-A-35 35 128 ist die katalytische Dealkoxilierung von geminalen Dialkoxiverbindungen zur Herstellung von Vinylethern an Zeolithen vom ZSM 5- und Mordenit-Typ, die ein bestimmtes $Na_2O/Al_2O_3$-Verhältnis von 1:1,05 (±0,25) aufweisen, bekannt. Mit diesen Katalysatoren wird zum Beispiel bei der Synthese von Methoxipropen aus Acetondimethylacetal bei 93 % Umsatz eine Selektivität von 90 % erreicht.

Aus der DE-A-37 22 891 ist ein Verfahren zur Herstellung von Vinylethern aus Acetalen an Boro- und/oder Eisensilikatzeolithen in ihren aciden H-Formen bekannt. Diese Katalysatoren desaktivieren jedoch binnen weniger Stunden durch Verkokung und erzwingen so häufige Unterbrechungen des Produktionsvorgangs zur Regenerierung des Katalysators.

Aus der DE-A-38 04 162 ist ein Verfahren zur Herstellung von Enolethern aus Acetalen unter Verwendung von Phosphaten mit Zeolithstruktur, gefällten Phosphaten der Elemente Bor, Zirkon, Cer, Eisen, Borsäure oder Phosphorsäure auf Trägermaterial und/oder sauren undotierten Metalloxiden als Katalysatoren bekannt. All diese stark sauren Katalysatoren zeigen zwar hohe Selektivitäten, aber auch ihre Standzeit von wenigen Stunden sind für einen wirtschaftlichen Betrieb des Prozesses nicht ausreichend.

Aus Chemical Abstracts, Vol. 94(19);156 241 f ist ein Verfahren zur Herstellung von 2-Methoxipropen durch Umsetzung von 2,2-Dimethoxipropan an nicht acidem $\alpha$-Aluminiumoxid der Firma Alcoa mit einer geringen Porosität von geschätzt ca. 10 bis 15% bekannt. Dieser Katalysator erreicht bei 500°C erst 90 % Umsatz und desaktiviert rasch binnen weniger Stunden. Mit hochporösem acidem Aluminiumoxid ("saure Tonerde") vom nicht weiter spezifizierten Typ PS 100 (ohne Herstellerangabe) entsteht ausschließlich Aceton/Methanol bzw. das Folgeprodukt Hexamethylbenzol.

Der vorliegenden Erfindung lag daher die Aufgabe zugrunde, den zuvor genannten Nachteilen abzuhelfen.

Demgemäß wurde ein neues und verbessertes Verfahren zur Herstellung von Enolethern der allgemeinen Formel I

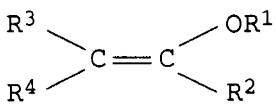

(I),

in der

| | |
|---|---|
| $R^1,R^2,R^3,R^4,R^5$ | $C_1$- bis $C_{20}$-Alkyl, $C_2$- bis $C_{20}$-Alkenyl oder $C_7$- bis $C_{20}$-Phenylalkyl, |
| $R^2,R^3,R^4$ | Wasserstoff, Aryl, Cyano, $-COOR^5$, durch $-COOR^5$, -C=O, Cyano, $C_1$- bis $C_{12}$-Alkoxy ein- bis dreifach substituiertes oder unterbrochenes $C_1$- bis $C_{12}$-Alkyl, durch $C_1$- bis $C_{12}$-Alkyl, $C_1$- bis $C_{12}$-Alkoxy, Halogen, Cyano ein- bis dreifach substituiertes Aryl oder $C_7$- bis $C_{20}$-Phenylalkyl und |
| $R^2$ und $R^4$ oder $R^3$ und $R^4$ | gemeinsam eine $C_3$- bis $C_{10}$-Alkylenkette oder gemeinsam eine $C_2$- bis $C_{20}$-Alkylidenkette |

bedeuten, aus Acetalen oder Ketalen der allgemeinen Formel II

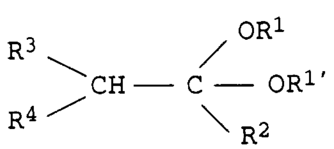

(II),

in der $R^1$, $R^2$, $R^3$ und $R^4$ die obengenannten und $R^{1'}$ unabhängig von $R^1$ dieselben Bedeutungen haben, in der Gasphase bei Temperaturen von 100 bis 550°C und einem Druck von 0,001 bis 5 bar an Heterogenkatalysatoren gefunden, welches dadurch gekennzeichnet ist, daß man als Heterogenkatalysatoren hochporöse Oxide der II., III. und IV. Hauptgruppe oder der II. und IV. Nebengruppe des Periodensystems der Elemente oder deren Gemische mit einem pH-Wert entsprechend 7 bis 14, einer Porosität von 40 bis 80% und einer BET-Oberfläche von 0,5 bis 250 $m^2/g$ einsetzt.

Das erfindungsgemäße Verfahren kann wie folgt durchgeführt werden:

Das Acetal bzw. das Ketal II kann durch Inkontaktbringen mit einem hochporösen, oxidischen Heterogenkatalysator im Festbett, in einer Wirbelschicht oder davon abgeleiteten Reaktorformen, z.B. einem Riser-Reaktor, bei Temperaturen von 100 bis 550°C, bevorzugt 180 bis 400°C, besonders bevorzugt 200 bis 350°C und einem Druck von 0,001 bis 5 bar, bevorzugt 0,01 bis 2 bar, besonders bevorzugt 0,1 bis 1 bar, insbesondere bei Normaldruck (Atmosphärendruck) und gegebenenfalls in Gegenwart eines Inertgases wie Stickstoff, Argon oder deren Gemische durchgeführt werden.

Das Verfahren kann so durchgeführt werden, daß man die auf Reaktionstemperatur erhitzten Ausgangsstoffe II über den auf Reaktionstemperatur erhitzten Katalysator leitet. Das den Reaktionsraum verlassende Gemisch kann kondensiert und fraktioniert destilliert werden.

Wird ein besonders hoher Umsatz angestrebt oder soll wenig oder kein Edukt (Ketal oder Acetal) im anfallenden Reaktoraustrag vorhanden sein, kann eventuell anfallendes Nebenprodukt, in der Regel das entsprechende Keton und eine erhöhte Menge an Alkohol, bei der nachfolgenden Destillation abgetrennt und, wenn gewünscht, erneut zu Edukt ketalisiert oder acetalisiert werden. Wird eine besonders hohe Selektivität angestrebt und soll folglich wenig oder kein Nebenprodukt (Keton oder Aldehyd) im anfallenden Reaktoraustrag vorhanden sein, kann eventuell anfallendes Edukt (Ketal oder Acetal) bei der nachfolgenden Destillation abgetrennt und, wenn gewünscht, erneut als Edukt eingesetzt werden.

Als hochporöse, oxidische Heterogenkatalysatoren eignen sich nicht acide Oxide der II., III. und IV. Hauptgruppe sowie die Oxide der II. und IV. Nebengruppe des Periodensystems der Elemente und deren Mischungen, bevorzugt Magnesiumoxid, Aluminiumoxid, Siliciumdioxid, Zinkoxid und Titandioxid und deren Mischungen, besonders bevorzugt $\alpha$-Aluminiumoxid, $\gamma$-Aluminiumoxid, Siliciumdioxid und Zinkoxid/$\gamma$-Aluminiumoxid und insbesondere $\alpha$-Aluminiumoxid mit einer Porosität in der Regel 40 bis 80%, bevorzugt 50 bis 75% und besonders bevorzugt 60 bis 70%.

Die Porosität ist definiert als das Verhältnis des Volumens der Poren eines Feststoffs zum Gesamtvolumen, also dem Volumen der Poren plus dem Volumen des Feststoffgerüsts.

Die Porosität kann zum Beispiel wie folgt bestimmt werden:

In ein Wägeschiffchen werden zwischen 15 und 20 Gramm der Probe des porösen Feststoffs eingewogen. Das so ermittelte Probengewicht in Gramm wird als $m_1$ bezeichnet. Diese Probe wird ein einen geeigneten evakuierbaren Kolben mit Vakuumanschluß und aufgesetztem Tropftrichter ohne Druckausgleich, z.B. einen Zweihalskolben (Rundkolben oder Spitzkolben) oder einen sogenannten Schlenkkolben, also einen Rundkolben mit einem absperrbaren Gas/Vakuumanschluß, der mit einem Tropftrichter ohne Druckausgleich versehen wurde, überführt und ca. 5 Minuten mit einer Vakuumpumpe auf 1 Torr Druck oder weniger evakuiert. Der Tropftrichter ist während des Evakuierens abgesperrt und wurde vorher mit soviel destilliertem Wasser gefüllt, daß nach der Evakuierphase die Probe im evakuierten Kolben komplett unter Wasser gestellt werden kann. Der Kolben wird anschließend vollständig belüftet und die Probe für eine Minute unter Wasser stehen gelassen. Anschließend wird die Probe vom überschüssigen Wasser getrennt, zum Beispiel durch ein geeignetes Sieb oder einen Filter. Bei porösen Filtermedien, zum Beispiel Filterpapier oder Glasfritten, ist darauf zu achten, daß sie angefeuchtet sind, da sonst der Probe wieder Wasser entzogen wird. Danach ist die Probe von anhaftenden Wassertropfen zu befreien, zum Beispiel durch Abstreifen der Formkörper auf einem angefeuchteten Filterpapier in einer Petrischale oder durch Aufstoßen des Siebes oder des Filters auf eine feste Unterlage.

Die Probe wird nun in ein Pyknometer geeigneten Volumens V in Milliliter mit der Masse $m_2$ in Gramm überführt und anschließend wird das Gesamtgewicht des Pyknometers mit der Probe bestimmt. Dieses Gesamtgewicht in Gramm wird als $m_3$ bezeichnet. Das Pyknometer wird nun mit Wasser aufgefüllt. Bei bestimmten Formkörpern, zum Beispiel Ringen, können sich an bestimmten Stellen der Formkörper, bei Ringen zum Beispiel im inneren Hohlraum der Ringe, Luftblasen bilden. Diese Luftblasen sind zu entfernen, zum Beispiel durch vorsichtiges Aufstoßen des Pyknometers auf eine harte Unterlage. Das Gewicht des mit Probe und Wasser gefüllten Pyknometers in Gramm wird bestimmt und als $m_4$ bezeichnet.

Die Porosität in Prozent errechnet sich daraus wie folgt:

$$Porosität = \frac{m_3 - m_2 - m_1}{(V - (m_4 - m_3))\ 100}\ [\%]$$

Die Acidität der hochporösen, oxidischen Heterogenkatalysatoren liegt im allgemeinen bei einem pH-Wert von 7 bis 14, bevorzugt 8 bis 14, besonders bevorzugt 11 bis 14. Für $\gamma$-Aluminiumoxid und Siliciumdioxid liegt diese Acidität vorteilhafterweise bei einem von pH-Wert von 11 bis 14 und für Zinkoxid und $\alpha$-Aluminiumoxid vorteilhafterweise bei einem von pH-Wert von 7 bis 12, bevorzugt 8 bis 11.

Die Acidität kann wie folgt geprüft werden:

10 g des Katalysators werden gemahlen und in 100 ml destilliertem oder vollentsalztem Wasser mit pH-Wert 7 suspendiert. Anschließend wird der pH-Wert der Suspension gemessen, bis ein stabiler Wert erreicht wird. Das Erreichen eines stabilen pH-Wertes kann etwas Zeit, etwa bis zu einer Stunde, in Anspruch nehmen.

Die BET-Oberfläche [Chem. Ing. Techn. **35** (1963) Seite 586 bis 589] der hochporösen, oxidischen Heterogenkatalysatoren liegt in der Regel bei 0,5 bis 250 $m^2/g$, bevorzugt 0,5 bis 200 $m^2/g$, besonders bevorzugt 0,6 bis 50 $m^2/g$.

Diese Katalysatoren können nach allgemein bekannten Verfahren hergestellt werden, zum Beispiel durch Fällung

geeigneter löslicher Vorläuferverbindungen oder von Gemischen geeigneter löslicher Vorläuferverbindungen und anschließende Trocknung und/oder Kalzinierung. Mischoxidkatalysatoren können zum Beispiel durch gemeinsame Fällung aus einer Lösung, die alle Vorläuferkomponenten in gelöster Form enthält, hergestellt werden, durch Auffällung oder Auftränkung gelöster Komponenten auf einen Festkörper, der andere Komponenten des Mischoxids enthält und anschließende Trocknung und/oder Calcinierung, oder auch durch eine physikalische Mischung geeigneter fester Vorläuferkomponenten. Zur Einstellung der Porosität können an geeigneter Stelle Hilfsstoffe, zum Beispiel Ausbrennstoffe, die beim Calcinieren unter Ausbildung eines bestimmten Porengefüges zersetzt und zu gasförmigen Verbindungen umgewandelt werden, zugegeben werden. Beim Prozeß der Katalysatorherstellung können weitere, allgemein bekannte Verfahrensschritte durchlaufen werden, wie zum Beispiel weitere Trocknungs-, Calcinierungs-, Tränk-, Reinigungs- oder Formgebungsschritte. Die physikalische Form des Katalysators kann den Erfordernissen der Reaktionsführung und den Gegebenheiten des vorhandenen Reaktors angepaßt werden. Die hochporösen, oxidischen Katalysatoren können als Formkörper, zum Beispiel Extrudate, Tabletten, Ringe, Kugeln, Berl-Sättel, in der Form der von der Firma Rauscher angebotenen Hi-Flow-Formkörper, mit mehreren parallelen Durchgängen versehene Tabletten oder in jeder anderen geeigneten Form eingesetzt werden.

Zahlreiche geeignete Oxide sind kommerziell erhältlich und werden häufig als Katalysatorträger angeboten.

Katalysatoren, die im Originalzustand zu hohe Aciditäten aufweisen, wie zum Beispiel die meisten kommerziell erhältlichen $\gamma$-Aluminiumoxide und Siliciumdioxide mit der geforderten Porosität und BET-Oberfläche können durch Zusatz alkalischer Verbindungen, wie z.B. Natriumhydroxid, Triethylamin, Hexamethylendiamin usw., auf die gewünschte Acidität eingestellt werden. Der Zusatz an alkalischer Verbindung kann zum Beispiel durch die bekannten Verfahren des Ionenaustauschs oder durch Imprägnierung des zu aciden Katalysators mit einer alkalischen Verbindung oder einer Lösung davon in einem geeigneten Lösungsmittel, zum Beispiel durch Imprägnierung mit einer wäßrigen Natriumhydroxidlösung oder auch durch eine andere Lösung einer Verbindung eines Alkali- oder Erdalkalielementes, eines organischen Amins oder einer anderen geeigneten Verbindung und, falls zweckmäßig, anschließende Trocknung, z.B. bei einer Temperatur von 20 bis 150°C und/oder Calcinierung, z.B. bei einer Temperatur von 150 bis 1500°C erfolgen.

Im Normalfall ist jedoch ein Katalysator aus $\alpha$-Aluminiumoxid mit einer Porosität oberhalb 60 %, einer Acidität entsprechend einem pH-Wert von mindestens 7 und einer BET-Oberfläche von mindestens 0,5 $m^2/g$ ein besonders bevorzugter Katalysator, um die Reaktion bei praktisch vollständigem Umsatz und nahezu 100%-iger Selektivität bei Temperaturen von 200 bis 350°C unter Normaldruck oder reduziertem Druck durchzuführen.

Die Substituenten $R^1$, $R^{1'}$, $R^2$, $R^3$, $R^4$ und $R^5$ in den Verbindungen I und II haben folgende Bedeutungen:

$R^1$,$R^{1'}$,$R^2$,$R^3$,$R^4$,$R^5$ unabhängig voneinander, bevorzugt $R^1 = R^{1'}$,

- $C_1$- bis $C_{20}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt $C_1$- bis $C_4$-Alkyl wie Methyl, Ethyl, n-Propyl, iso-Propyl, n-Butyl, iso-Butyl, sec.-Butyl und tert.-Butyl, besonders bevorzugt $C_1$- bis $C_3$-Alkyl wie Methyl, Ethyl, n-Propyl und iso-Propyl,
- $C_7$- bis $C_{20}$-Phenylalkyl, bevorzugt $C_7$- bis $C_{12}$-Phenylalkyl wie Benzyl, 1-Phenethyl, 2-Phenethyl, 1-Phenylpropyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- $C_2$- bis $C_{20}$-Alkenyl, wie Vinyl, Propenyl, iso-Propenyl und Butenyl,

$R^2$,$R^3$,$R^4$

- Wasserstoff,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- Cyano,
- -$COOR^5$,
- durch -$COOR^5$, -C=O, Cyano, $C_1$- bis $C_{12}$-Alkoxy ein- bis dreifach substituiertes oder unterbrochenes $C_1$- bis $C_{12}$-Alkyl wie Essigsäuremethylester, Essigsäureethylester, Acetyl, Cyanomethyl, Cyanoethyl, Methoxymethyl, Methoxyethyl, Ethoxymethyl, Ethoxyethyl, Methoxypropyl und Ethoxypropyl,
- durch $C_1$- bis $C_{12}$-Alkyl, $C_1$- bis $C_{12}$-Alkoxy, Halogen, Cyano ein- bis dreifach substituiertes Aryl wie o-Chlorphenyl, p-Chlorphenyl, 2,4-Dichlorphenyl, o-Cyanophenyl, p-Cyanophenyl, 2,4-Dicyanophenyl, o-Methoxyphenyl, p-Methoxyphenyl, o-Ethoxyphenyl und p-Ethoxyphenyl,
- durch $C_1$- bis $C_{12}$-Alkyl, $C_1$- bis $C_{12}$-Alkoxy, Halogen, Cyano ein- bis dreifach substituiertes $C_7$- bis $C_{20}$-Phenylalkyl wie o-Chlorbenzyl, p-Chlorbenzyl, 2,4-Dichlorbenzyl, o-Cyanobenzyl, p-Cyanobenzyl, 2,4-Dicyanobenzyl, o-Methoxybenzyl, p-Methoxybenzyl, o-Ethoxybenzyl und p-Ethoxybenzyl, o-Chlorphenylethyl, p-Chlorphenylethyl, 2,4-Dichlorphenylethyl, o-Cyanophenylethyl, p-Cyanophenylethyl, 2,4-Dicyanophenylethyl, o-Methoxyphenylethyl, p-Methoxyphenylethyl, o-Ethoxyphenylethyl und p-Ethoxyphenylethyl und

$R^2$ und $R^4$ oder $R^3$ und $R^4$ gemeinsam eine

- $C_3$- bis $C_{10}$-Alkylenkette wie $(CH_2)_3$, $(CH_2)_4$, $(CH_2)_5$, $(CH_2)_6$, $(CH_2)_7$, $(CH_2)_8$, $(CH_2)_9$ und $(CH_2)_{10}$, bevorzugt $(CH_2)_3$, $(CH_2)_4$, $(CH_2)_5$ und $(CH_2)_6$, besonders bevorzugt $(CH_2)_3$, $(CH_2)_4$ und $(CH_2)_6$,

$R^3$ und $R^4$

- gemeinsam eine $C_2$- bis $C_{20}$-Alkylidenkette wie Ethyliden, Propyliden und iso-Propyliden.

Als Ausgangsverbindungen II eignen sich zum Beispiel 1,1-Dimethoxiethan, 1,1-Dimethoxipropan, 2,2-Dimethoxipropan, 1,1-Dimethoxibutan, 2,2-Dimethoxipropionsäuremethylester, 3,3-Dimethoxipropionsäuremethylester, 1,1-Diethoxiethan und 1,1-Diethoxipropan.

Enolether, und speziell Vinylether werden bei der Herstellung bestimmter Homo- und Copolymerisate eingesetzt, die Anwendung auf den Gebieten der Lack- und Klebstoffherstellung sowie als Hilfsmittel in der Textil- und Lederindustrie finden. Weiterhin dienen Enolether als wertvolle Zwischenprodukte für organische Synthesen, z. B. für Diels-Alder-Reaktionen, für die Herstellung von Glutardialdehyden, $\gamma$-Pyran und $\gamma$-Picolin sowie von Wirkstoffen.

Beispiele

Tabelle 1

| eingesetzte Katalysatoren | | | | |
|---|---|---|---|---|
| Katalysator | Zusammensetzung | Porosität [%] | BET-Oberfläche [$m^2/g$] | pH-Wert |
| A | 90 Gew.-% ZnO, 10 Gew.-% $\gamma$-$Al_2O_3$ | 54 | 60 | 10,61 |
| B | >99 Gew.-% $\alpha$-$Al_2O_3$ | 69,2 | 0,9 | 8,23 |
| C | >99 Gew.-% $SiO_2$ | 67,1 | 140 | 5,58 |
| D | >99 Gew.-% $\gamma$-$Al_2O_3$ | 66,1 | 180 | 8,67 |
| E | >99 Gew.-% $\alpha$-$Al_2O_3$ | 68,2 | 1,2 | 8,52 |
| F | >99 Gew.-% $\alpha$-$Al_2O_3$ | 63,6 | 4,1 | 10,33 |
| G | >99 Gew.-% $\alpha$-$Al_2O_3$ | 62,7 | 0,5 | 9,93 |

Beispiel 1

Stündlich wurden 30 ml 2,2-Dimethoxipropan verdampft und durch einen auf 300°C erhitzten Festbettreaktor, der mit 60 ml des Katalysators A gefüllt war, geleitet. Die Reaktionsgase wurden anschließend kondensiert und destilliert. Man erhielt stündlich 16,9 g (96%) 2-Methoxipropen. Es wurde keine Katalysator-Desaktivierung beobachtet.

Beispiel 2

Stündlich wurden 60 ml 2,2-Dimethoxipropan verdampft und durch einen auf 220°C erhitzten Festbettreaktor, der mit 30 ml des Katalysators B gefüllt war, geleitet. Die Reaktionsgase wurden anschließend kondensiert und destilliert. Man erhielt stündlich 34,2 g (97%) 2-Methoxipropen. Es wurde keine Katalysator-Desaktivierung beobachtet.

Beispiel 3

Stündlich wurden 480 ml 2,2-Dimethoxipropan verdampft und durch einen auf 300°C erhitzten Festbettreaktor, der mit 60 ml des Katalysators B gefüllt war, geleitet. Die Reaktionsgase wurden anschließend kondensiert und destilliert.

Man erhielt stündlich 265,2 g (94%) 2-Methoxipropen. Es wurde keine Katalysator-Desaktivierung beobachtet. Das Beispiel zeigte die hohe Belastbarkeit des Katalysators B.

Beispiel 4

Stündlich wurden 15 ml 2,2-Dimethoxipropan verdampft und durch einen auf 300°C erhitzten Festbettreaktor, der mit 60 ml des Katalysators C gefüllt war, geleitet. Die Reaktionsgase wurden anschließend kondensiert und gaschromatographisch analysiert. Der Reaktoraustrag enthielt 22 Fl.-% Methanol, 45 Fl.-% Aceton und 2 Fl.-% 2-Methoxipropen.

"Fl.-%" sind hier und im folgenden die Flächenprozente, die sich nach Integration der Gaschromatogramme, die zur Analyse des Reaktoraustrags dienten, errechneten. Das Beispiel zeigte, daß der unbehandelte Katalysator C unter diesen Bedingungen bei vollständigem Umsatz (kein 2,2-Dimethoxipropan nachweisbar) nur sehr geringe Selektivität zum gewünschten Wertprodukt 2-Methoxipropen zeigt. Stattdessen wird das Ketal fast vollständig zum Keton und Alkohol abgebaut. Es wurde keine Katalysator-Desaktivierung beobachtet.

Beispiele 5 bis 8

Der Siliciumdioxidkatalysator C aus dem jeweils vorigen Versuch wurde ausgebaut, in 100 ml Wasser suspendiert, mit 10 ml einer wäßrigen Lösung, welche jeweils die in der folgenden Tabelle angegebene Menge Natriumhydroxid enthielt, versetzt, nach zwei Stunden Tränkdauer am Rotationsverdampfer bei 100°C im Wasserstrahlvakuum getrocknet und im jeweils nächsten Versuch eingesetzt.

Die in der Tabelle 2 zusammengefaßten Beispiele 5 bis 8 wurden analog zu Beispiel 4 durchgeführt. Stündlich wurden 30 ml 2,2-Dimethoxipropan verdampft. In keinem Fall wurde Katalysator-Desaktivierung beobachtet.

Tabelle 2

| Beispiel Nr. | NaOH [mg] | pH-Wert | Methanol [Fl.-%] | Aceton [Fl.-%] | 2-Methoxipropen [Fl.-%] | 2,2-Dimethoxipropan [Fl.-%] |
|---|---|---|---|---|---|---|
| 5 | 133 | 8,95 | 25 | 32 | 7 | 0 |
| 6 | 713 | 10,43 | 20 | 13 | 52 | 0 |
| 7 | 845 | 11,50 | 20 | 4 | 73 | 1 |
| 8 | 1706 | 12,22 | 17 | 1 | 65 | 16 |

Die Beispiele 5 bis 8 zeigen, daß der im Originalzustand unselektive Katalysator C durch Behandlung mit Alkali selektiver wird: Der Anteil des gewünschten Produkts 2-Methoxipropen am Produktstrom steigt stetig mit dem pH-Wert und der Anteil des Nebenprodukts Aceton sinkt stetig. Beispiel 8 zeigt, daß zunehmende Alkalibehandlung die Aktivität des Katalysators senkt, da nicht umgesetztes Edukt in signifikanten Anteilen detektiert wird.

Beispiel 9

Stündlich wurden 30 ml 2,2-Dimethoxipropan verdampft und durch einen auf 300°C erhitzten Festbettreaktor, der mit 60 ml des Katalysators D gefüllt war, geleitet. Die Reaktionsgase wurden anschließend kondensiert und gaschromatographisch analysiert. Der Reaktoraustrag enthielt 12 Fl.-% Methanol und 74 Fl.-% Aceton. Das Beispiel zeigt, daß der unbehandelte Katalysator D unter diesen Bedingungen bei vollständigem Umsatz (kein 2,2-Dimethoxipropan nachweisbar) keine Selektivität zum gewünschten Wertprodukt 2-Methoxipropen zeigte. Stattdessen wurde das Ketal fast vollständig zum Keton und dem Alkohol abgebaut. Es wurde keine Katalysator-Desaktivierung beobachtet.

Beispiele 10 bis 12

Der $\gamma$-Aluminiumoxidkatalysator D aus dem jeweils vorigen Beispiel wurde ausgebaut, in 100 ml Wasser suspendiert, mit 10 ml einer wäßrigen Lösung, welche jeweils die in der Tabelle 3 angegebene Menge Natriumhydroxid enthielt, versetzt, nach zwei Stunden am Rotationsverdampfer bei 100°C im Wasserstrahlvakuum getrocknet und im jeweils nächsten Versuch eingesetzt.

Die Beispiele 10 bis 12 wurden analog zu Beispiel 9 durchgeführt. Stündlich wurden 30 ml 2,2-Dimethoxipropan verdampft. In keinem Fall wurde Katalysator-Desaktivierung beobachtet.

Tabelle 3

| Beispiel Nr. | NaOH [mg] | pH-Wert | Methanol [Fl.-%] | Aceton [Fl.-%] | 2-Methoxipropen [Fl.-%] | 2,2-Dimethoxipropan [Fl.-%] |
|---|---|---|---|---|---|---|
| 10 | 133 | 10,12 | 23 | 41 | 20 | 0 |
| 11 | 713 | 12,01 | 20 | 9 | 68 | 0 |
| 12 | 845 | 12,57 | 17 | 1 | 69 | 12 |

Die Beispiele 10 bis 12 zeigen, daß der im Originalzustand unselektive Katalysator D durch Behandlung mit Alkali selektiver wird: Der Anteil des gewünschten Produkts 2-Methoxipropen am Produktstrom steigt stetig mit dem pH-Wert und der Anteil des Nebenprodukts Aceton sinkt stetig. Beispiel 12 zeigt, daß zunehmende Alkalibehandlung die Aktivität des Katalysators senkt, da unumgesetztes Edukt in signifikanten Anteilen detektiert wird.

Beispiel 13

Stündlich wurden 60 ml 1,1-Diethoxipropan verdampft und durch einen auf 300°C erhitzten Festbettreaktor, der mit 60 ml des Katalysators E gefüllt war, geleitet. Die Reaktionsgase wurden anschließend kondensiert und gaschromatographisch analysiert. Der Reaktoraustrag enthielt 26 Fl.-% Ethanol, 64 Fl.-% cis/trans 1-Ethoxipropen und 1 Fl.-% 1,1-Diethoxipropan. Es wurde keine Katalysator-Desaktivierung beobachtet.

Beispiel 14

Stündlich wurden 60 ml 2,2-Dimethoxipropionsäuremethylester verdampft und durch einen auf 250°C erhitzten Festbettreaktor, der mit 60 ml des Katalysators E gefüllt war, geleitet. Die Reaktionsgase wurden anschließend kondensiert und gaschromatographisch analysiert. Der Reaktoraustrag enthielt 14 Fl.-% Methanol, 82 Fl.-% 2-Methoxiacrylsäuremethylester und 1 Fl.-% 2,2-Dimethoxipropionsäuremethylester. Es wurde keine Katalysator-Desaktivierung beobachtet.

Beispiel 15

Stündlich wurden 30 ml 2,2-Dimethoxipropan verdampft und durch einen auf 300°C erhitzten Festbettreaktor, der mit 60 ml des Katalysators F gefüllt war, geleitet. Die Reaktionsgase wurden anschließend kondensiert und gaschromatographisch analysiert. Der Reaktoraustrag enthielt 20 Fl.-% Methanol, 1 Fl.-% Aceton, 77 Fl.-% 2-Methoxipropen und 1 Fl.-% 2,2-Dimethoxipropan. Es wurde keine Katalysator-Desaktivierung beobachtet.

Beispiel 16

Stündlich wurden 30 ml 2,2-Dimethoxipropan verdampft und in Sumpffahrweise durch einen auf 300°C erhitzten Festbettreaktor, der mit 60 ml des Katalysators G gefüllt war, geleitet. Die Reaktionsgase wurden anschließend kondensiert und gaschromatographisch analysiert. Der Reaktoraustrag enthielt 14 Fl.-% Methanol, 2 Fl.-% Aceton, 52 Fl.-% 2-Methoxipropen und 32 Fl.-% 2,2-Dimethoxipropan.

Die Beispiele 13, 14 und 15 zeigen, daß verschiedene geeignete Edukte an verschiedenen erfindungsgemäßen Katalysatoren bei vollständigem oder fast vollständigem Umsatz und einer Selektivität von oder nahe bei 100 % zu den gewünschten Enolethern umgesetzt werden können. Beispiel 16 zeigt, daß ein Katalysator aus $\alpha$-Aluminiumoxid mit einer BET-Oberfläche von 0,5 $m^2/g$ im Vergleich zu einem Katalysator aus $\alpha$-Aluminiumoxid mit einer BET-Oberfläche von 0,9 $m^2/g$ (Katalysator B) oder höher BET-Oberflächen (Katalysatoren E und F) einen geringeren Umsatz aufweist, also inaktiver ist.

Beispiel 17

Stündlich wurden 10 ml 1,1-Dimethoxy-3-methyl-but-2-en verdampft und durch einen auf 350°C erhitzten Festbettreaktor, der mit 60 ml des Katalysators B gefüllt war, geleitet. Die Reaktionsgase wurden anschließend kondensiert und gaschromatographisch analysiert. Der Reaktoraustrag enthielt 10,3 Fl.-% Methanol und 84,0 Fl.-% 1-Methoxy-3-methyl-1,3-butadien (cis/trans-Gemisch).

Beispiel 18

2,2-Dimethoxy-4-methyl-3-penten (56 g, ca. 90 %ig) wurden bei 30 mbar innerhalb von 3 Stunden durch einen auf 250°C erhitzten und mit 35 ml Katalysator B gefüllten Festbettreaktor geleitet. Der Reaktoraustrag wurde in einer Kühlfalle kondensiert und anschließend unter Zusatz eines KOH-Plätzchens destilliert. Die Fraktion, die bei 48-52°C (50 mbar) siedete (insgesamt 41,5 g), enthielt 44 % 2-Methoxy-4-methyl-pentadien-1,3, 37 % 2-Methyl-4-methoxy-pentadien-1,3 (als cis/trans-Gemisch), 4 % 2-Methoxy-4-methyl-pentadien-1,4, 5 % Ausgangsprodukt und 8 % Mesityloxid, das als Verunreinigung im Ausgangsprodukt enthalten war.

Beispiel 19

2,2,4-Trimethoxy-4-methylpentan (70 g, ca. 95 %ig) wurde bei 30 mbar innerhalb von 3 Stunden durch einen auf 250°C erhitzen und mit 35 ml Katalysator B gefüllten Festbettreaktor geleitet. Der Reaktoraustrag wurde in einer Kühlfalle kondensiert und anschließend unter Zusatz eines KOH-Plätzchens bei Normaldruck destilliert, um das gebildete Methanol zu entfernen. Das Rohprodukt enthielt 23 % 2-Methoxy-4-methyl-pentadien-1,3, 29 % 2-Methyl-4-methoxy-pentadien-1,3 (als cis/trans-Gemisch), 7 % 2-Methoxy-4-methyl-pentadien-1,4 und 35 % 2,4-Dimethoxy-4-methyl-1-penten.

Beispiel 20

1,1-Dimethoxyhexan (158 g) wurde bei 25 mbar innerhalb von 4,5 Stunden durch einen auf 350°C erhitzten und mit 35 ml B gefüllten Festbettreaktor geleitet. Der Reaktoraustrag wurde in einer Kühlfalle kondensiert und anschließend unter Zusatz eines KOH-Plätzchens destilliert. 1-Methoxy-1-hexen (111 g, cis/trans-Gemisch) wurde als farblose Flüssigkeit (Kp. 52°C/60 mbar) in einer Ausbeute von 90 % erhalten.

Beispiel 21

1,1-Dimethoxyoctan (209 g) wurde bei 25 mbar innerhalb von 4 Stunden durch einen auf 350°C erhitzten und mit 35 ml Katalysator B gefüllten Festbettreaktor geleitet. Der Reaktoraustrag wurde in einer Kühlfalle kondensiert und anschließend unter Zusatz eines KOH-Plätzchens destilliert. 1-Methoxy-1-octen (164 g, cis/trans-Gemisch) wurde als farblose Flüssigkeit (Kp. 78°C/30 mbar) in einer Ausbeute von 96 % erhalten.

Beispiel 22

1,1-Dimethoxydecan (110 g) wurde bei 15 mbar innerhalb von 4 Stunden durch einen auf 350°C erhitzten und mit 35 ml Katalysator B gefüllten Festbettreaktor geleitet. Der Reaktoraustrag wurde in einer Kühlfalle kondensiert. Nach dem Auftauen bildeten sich zwei Phasen. Die methanolische Phase wurde abgetrennt und die andere Phase unter Zusatz eines KOH-Plätzchens destilliert. 1-Methoxy-1-decen (68 g, cis/trans-Gemisch) wurde als farblose Flüssigkeit (Kp. 80°C/1 mbar) in einer Ausbeute von 74 % erhalten.

Beispiel 23

1,1-Dimethoxydodecan (95 g) wurde innerhalb von 4 Stunden bei 12 mbar durch einen auf 350°C erhitzten und mit 35 ml Katalysator B gefüllten Festbettreaktor geleitet. Der Reaktoraustrag wurde in einer Kühlfalle kondensiert. Nach dem Auftauen bildeten sich zwei Phasen. Die methanolische Phase wurde abgetrennt und die andere Phase unter Zusatz eines KOH-Plätzchens destilliert. 1-Methoxy-1-dodecen (58 g, cis/trans-Gemisch) wurde als farblose Flüssigkeit (Kp. 100°C/1 mbar) in einer Ausbeute von 72 % erhalten.

**Patentansprüche**

1. Verfahren zur Herstellung von Enolethern der allgemeinen Formel I

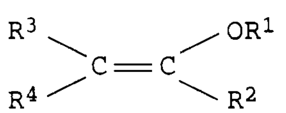

(I),

in der

| | |
|---|---|
| $R^1,R^2,R^3,R^4,R^5$ | $C_1$- bis $C_{20}$-Alkyl, $C_2$- bis $C_{20}$-Alkenyl oder $C_7$- bis $C_{20}$-Phenylalkyl, |
| $R^2,R^3,R^4$ | Wasserstoff, Aryl, Cyano, $-COOR^5$, durch $-COOR^5$, -C=O, Cyano, $C_1$- bis $C_{12}$-Alkoxy ein- bis dreifach substituiertes oder unterbrochenes $C_1$- bis $C_{12}$-Alkyl, durch $C_1$- bis $C_{12}$-Alkyl, $C_1$- bis $C_{12}$-Alkoxy, Halogen, Cyano ein- bis dreifach substituiertes Aryl oder $C_7$- bis $C_{20}$-Phenylalkyl und |
| $R^2$ und $R^4$ oder $R^3$ und $R^4$ | gemeinsam eine $C_3$- bis $C_{10}$-Alkylenkette oder gemeinsam eine $C_2$- bis $C_{20}$-Alkylidenkette |

bedeuten, aus Acetalen oder Ketalen der allgemeinen Formel II

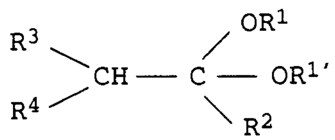

(II),

in der $R^1$, $R^2$, $R^3$ und $R^4$ die obengenannten und $R^{1'}$ unabhängig von $R^1$ dieselben Bedeutungen haben, in der Gasphase bei Temperaturen von 100 bis 550°C und einem Druck von 0,001 bis 5 bar an Heterogenkatalysatoren, dadurch gekennzeichnet, daß man als Heterogenkatalysatoren hochporöse Oxide der II., III. und IV. Hauptgruppe oder der II. und IV. Nebengruppe des Periodensystems der Elemente oder deren Gemische mit einem pH-Wert entsprechend 7 bis 14, einer Porosität von 40 bis 80% und einer BET-Oberfläche von 0,5 bis 250 $m^2/g$ einsetzt.

**2.** Verfahren zur Herstellung von Enolethern I nach Anspruch 1, dadurch gekennzeichnet, daß man die Umsetzung bei Temperaturen von 180 bis 400°C durchführt.

**3.** Verfahren zur Herstellung von Enolethern I nach Anspruch 1, dadurch gekennzeichnet, daß man die Umsetzung bei Temperaturen von 200 bis 350°C durchführt.

**4.** Verfahren zur Herstellung von Enolethern I nach den Ansprüchen 1 bis 3, dadurch gekennzeichnet, daß man als Heterogenkatalysatoren hochporöses Magnesiumoxid, Aluminiumoxid, Siliciumdioxid, Zinkoxid und Titandioxid oder deren Gemische einsetzt.

**5.** Verfahren zur Herstellung von Enolethern I nach den Ansprüchen 1 bis 4, dadurch gekennzeichnet, daß man als Heterogenkatalysatoren hochporöses $\alpha$-Aluminiumoxid, $\gamma$-Aluminiumoxid, Siliciumdioxid und Zinkoxid/$\gamma$-Aluminiumoxid oder deren Gemische einsetzt.

**6.** Verfahren zur Herstellung von Enolethern I nach den Ansprüchen 1 bis 5, dadurch gekennzeichnet, daß man als Heterogenkatalysatoren $\gamma$-Aluminiumoxid, Siliciumdioxid oder deren Gemische mit einem pH-Wert entsprechend 11 bis 14 einsetzt.

**7.** Verfahren zur Herstellung von Enolethern I nach den Ansprüchen 1 bis 5, dadurch gekennzeichnet, daß man als Heterogenkatalysatoren hochporöses $\alpha$-Aluminiumoxid einsetzt.

**8.** Verfahren zur Herstellung von Enolethern I nach den Ansprüchen 1 bis 7, dadurch gekennzeichnet, daß die Porosität der Heterogenkatalysatoren oberhalb 50% beträgt.

**9.** Verfahren zur Herstellung von Enolethern I nach den Ansprüchen 1 bis 8, dadurch gekennzeichnet, daß die Porosität der Heterogenkatalysatoren oberhalb 60% beträgt.

**10.** Verwendung der Katalysatoren nach Anspruch 1 bis 9 zur Herstellung von Enolethern I.

Europäisches Patentamt

# EUROPÄISCHER RECHERCHENBERICHT

Nummer der Anmeldung
EP 96 11 8612

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A<br>D,A | EP 0 327 985 A (BASF)<br>* Ansprüche; Beispiele *<br>& DE 38 04 162 A<br>--- | 1-10 | C07C41/28<br>C07C43/16 |
| A<br>D,A | EP 0 299 286 A (BASF)<br>* Ansprüche; Beispiele *<br>& DE 37 22 891 A<br>--- | 1-10 | |
| A<br>D,A | EP 0 217 089 A (DEGUSSA)<br>* Ansprüche; Beispiele *<br>& DE 35 35 128 A<br>--- | 1-10 | |
| A | GB 706 712 A (RUHRCHEMIE)<br>* das ganze Dokument *<br>--- | 1-10 | |
| A | JOURNAL OF CHEMICAL TECHNOLOGY AND BIOTECHNOLOGY,<br>Bd. 58, 1993, LONDON,<br>Seiten 211-214, XP000404018<br>L. CERVENY ET AL: "Catalytic splitting of acetals to unsaturated ethers"<br>* das ganze Dokument *<br>--- | 1-10 | RECHERCHIERTE SACHGEBIETE (Int.Cl.6) |
| D,A | CHEMICAL ABSTRACTS, vol. 94, no. 19, 11.Mai 1981<br>Columbus, Ohio, US;<br>abstract no. 156241f,<br>M. BANCIU ET AL: "A laboratory method for synthesis of isopropenyl methyl ether"<br>Seite 599; Spalte 2;<br>XP002025646<br>* Zusammenfassung *<br>& BUL. INST. POLITEH. "GHEORGHE GHEORGHIU-DEJ" BUCURESTI, SER. CHIM-METAL,<br>Bd. 42, Nr. 1,<br>Seiten 101-105,<br>----- | 1-10 | C07C |

Der vorliegende Recherchenbericht wurde für alle Patentansprüche erstellt

| Recherchenort | Abschlußdatum der Recherche | Prüfer |
|---|---|---|
| DEN HAAG | 19.Februar 1997 | Wright, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)